# EUROPÄISCHE PATENTSCHRIFT

(11) **EP 1 978 863 B1**
(45) Veröffentlichungstag und Bekanntmachung des Hinweises auf die Patenterteilung: **14.09.2011**
(21) Anmeldenummer: 06806594.5
(22) Anmeldetag: 28.10.2006
(51) Int. Cl.: A61B 5/00, A61B 5/03

(54) **VERFAHREN UND VORRICHTUNG ZUR ERFASSUNG VON PHYSIOLOGISCHEN MESSDATEN**
METHOD AND DEVICE FOR RECORDING PHYSIOLOGICAL MEASUREMENT DATA
PROCEDE ET DISPOSITIF POUR LA SAISIE DE DONNEES DE MESURE PHYSIOLOGIQUES

(30) Priorität: 01.02.2006 DE 102006004523
(43) Veröffentlichungstag der Anmeldung: 15.10.2008
(73) Patentinhaber: Aesculap AG, 78532 Tuttlingen (DE)
(72) Erfinder: LUTZE, Theodor, 78582 Balgheim (DE); SCHAUER, Dirk, 10318 Berlin (DE)
(74) Vertreter: Hoeger, Stellrecht & Partner Patentanwälte
(86) Internationale Anmeldenummer: PCT/EP2006/010387
(87) Internationale Veröffentlichungsnummer: WO 2007/087840

(56) Entgegenhaltungen:
- WO-A-97/17012
- DE-U1- 20 011 444
- DE-U1-202004 017 417
- DE-U1-202006 001 532

## Beschreibung

Die Erfindung betrifft ein Verfahren zur Datenerfassung von Meßdaten, die von einem physiologischen Sensor in Abhängigkeit von physiologischen Parametern eines Lebewesens erzeugt werden, bei dem die von dem physiologischen Sensor erzeugten Daten einer externen Auswerteeinheit zugeführt werden, die diese Daten speichert.

Außerdem betrifft die Erfindung eine Vorrichtung zur Datenerfassung von Meßdaten, die von einem physiologischen Sensor in Abhängigkeit von physiologischen Parametern eines Lebewesens erzeugt werden, mit einer Auswerteeinheit, die über eine Datenübertragungsstrecke mit dem physiologischen Sensor verbindbar ist, und mit einer Speichereinrichtung, in der die Auswerteeinheit die von dem physiologischen Sensor gelieferten Meßdaten speichert.

Zur Erfassung von physiologischen Parametern im Körper eines Lebewesens werden Sensoren verwendet, die entweder außerhalb des Körpers angeordnet werden oder die in den Körper implantiert sind und die in Abhängigkeit von physiologischen Parametern unterschiedliche elektrische Signale erzeugen, die als Meßdaten von einer externen Auswerteeinheit empfangen werden können. Die externe Auswerteeinheit steht dabei über eine Übertragungsstrecke mit dem jeweiligen physiologischen Sensor in Verbindung, es kann sich dabei um eine Übertragungsleitung handeln, es ist aber auch möglich, daß die Datenübertragung drahtlos mit einer der bekannten Übertragungstechniken erfolgt, also beispielsweise über Funksignale oder über optische Infrarotsignale.

Die Auswerteeinheit speichert üblicherweise die empfangenen Meßdaten, so daß aufgrund dieser Meßdaten die Überwachung der physiologischen Parameter des Körpers möglich ist, gegebenenfalls auch zu einem späteren Zeitpunkt (WO 97/17012).

Wenn die externe Auswerteeinheit mit verschiedenen physiologischen Sensoren zusammenarbeitet, müssen der Auswerteeinheit Daten eingegeben werden, die die gespeicherten Meßdaten jeweils dem physiologischen Sensor zuordnen, von dem sie stammen, mit anderen Worten, es müssen patientenspezifische Daten in die Auswerteeinheit eingegeben werden. Dies ist im Einzelfall kompliziert und kann zu Fehlern führen.

Es ist Aufgabe der Erfindung, ein gattungsgemäßes Verfahren so auszugestalten, daß in einfacher Weise eine sichere Zuordnung der von verschiedenen physiologischen Sensoren empfangenen Meßdaten zu dem jeweiligen physiologischen Sensor erfolgen kann.

Diese Aufgabe wird bei einem Verfahren der eingangs beschriebenen Art erfindungsgemäß durch die kennzeichnenden Merkmale von Anspruch 1 gelöst.

Durch das Vorsehen eines separaten Speichers, der nur einem einzigen physiologischen Sensor zugeordnet ist, ist sichergestellt, daß von diesem physiologischen Sensor stammende Daten immer nur in dem zugeordneten Speicher gespeichert werden, ohne daß noch zusätzliche patientenbezogene Daten eingegeben werden müssen.

Gemäß einer besonders vorteilhaften Ausgestaltung der Erfindung ist vorgesehen, daß die Auswerteeinheit zur Überprüfung der Zuordnung eines Speichers zu einem physiologischen Sensor Codierungsdaten, die vom physiologischen Sensor der Auswerteeinheit zugeführt werden, und Codierungsdaten, die jeweils in jedem Speicher gespeichert sind, miteinander vergleicht und die vom physiologischen Sensor gelieferten Meßdaten nur dann in einem Speicher speichert, wenn die Codierungsdaten des physiologischen Sensors und des Speichers zueinander passen. In diesem Falle kann ein akustisches und/optisches Warnsignal von der Auswerteeinheit abgegeben werden.

Auf diese Weise erhält man eine automatische Zuordnung des jeweiligen Speichers zu einem physiologischen Sensor aufgrund der Codierungsdaten, die einmal vom Sensor der Auswerteeinheit zugeführt werden und die zum anderen in dem zugeordneten Speicher gespeichert sind. Diese Codierungsdaten können beispielsweise eine Kennziffer oder eine Buchstabenfolge sein.

Es ist günstig, wenn die Codierungsdaten in dem Speicher unveränderbar gespeichert werden. Auf diese Weise ist ein Speicher dauerhaft einem bestimmten physiologischen Sensor zugeordnet, diese Zuordnung kann nicht aufgehoben werden.

Bei einer weiteren bevorzugten Ausführungsform ist vorgesehen, daß in den Speichern Kalibrationsdaten des zugeordneten physiologischen Sensors gespeichert werden und daß die Auswerteeinheit diese bei der Auswertung der Meßdaten berücksichtigt. Der Speicher "kennt" also bestimmte Kenngrößen des physiologischen Sensors und kann diese berücksichtigen, wenn die Meßdaten, die von dem physiologischen Sensor geliefert werden und die in dem zugeordneten Speicher gespeichert werden, ausgewertet werden. Es kann sich dabei z.B. um die Empfindlichkeit eines physiologischen Sensors handeln oder um eine verzögerte Ansprechzeit oder ähnliche Parameter. Da diese Daten in dem zugeordneten Speicher abgelegt sind, kann eine Verwechslung dieser Kalibrationsdaten bei Empfang von Meßdaten von einem anderen physiologischen Speicher nicht auftreten.

Besonders vorteilhaft ist es, wenn die verschiedenen, jeweils einem physiologischen Sensor zugeordneten Speicher als mobile Baueinheiten ausgebildet und diese zur Datenerfassung der Meßdaten eines physiologischen Sensors jeweils mit der Auswerteeinheit verbunden werden. Die Auswerteeinheit kann also alleine nicht die empfangenen Daten des physiologischen Sensors speichern, sondern sie benötigt dazu einen speziellen Speicher, der mit ihr verbunden wird. Eine Speicherung erfolgt nur dann, wenn dieser spezielle Speicher genau der dem physiologischen Sensor zugeordnete Speicher ist, von dem Daten empfangen werden.

Beispielsweise kann als Speicher eine Speicherkarte oder ein Wechselspeicher verwendet werden, insbesondere ein USB-Speichermodul oder eine Kompakt-Flash-Karte.

Bei einer weiteren Ausgestaltung der Erfindung ist vorgesehen, daß bei der Datenerfassung von Meßdaten des physiologischen Sensors zusätzlich mittels eines externen Sensors Meßdaten erzeugt werden, die Umgebungsparametern entsprechen, und daß diese Meßdaten zusammen mit den Meßdaten des physiologischen Sensors in dem diesem physiologischen Sensor zugeordneten Speicher gespeichert werden. Diese externen Parameter können beispielsweise Umgebungsdruck oder Umgebungstemperatur sein, so daß man bei der Auswertung diese externen Parameter mit den Meßdaten des physiologischen Sensors vergleichen kann.

Es kann vorgesehen sein, daß die Auswerteeinheit die von einem physiologischen Sensor der Auswerteeinheit zugeführten Daten nur dann in dem zugeordneten Speicher speichert, wenn diese Meßdaten bestimmte Plausibilitätskriterien erfüllen. Wenn die Meßdaten außerhalb eines vorbestimmten Wertebereichs liegen oder wenn der zeitliche Verlauf der Meßdaten deutlich von einem vorgegebenen zeitlichen Verlauf abweicht, kann dies ein Hinweis dafür sein, daß der physiologische Sensor nicht zuverlässig arbeitet, so daß dann eine Speicherung der Meßdaten unterbleibt. Statt dessen kann beispielsweise ein Warnsignal ausgegeben werden.

Insbesondere ist es günstig, wenn Vergleichsdaten für die Überprüfung der Plausibilitätskriterien in den den physiologischen Sensoren zugeordneten Speichern gespeichert werden.

Die physiologischen Sensoren können die unterschiedlichsten körpereigenen physiologischen Parameter bestimmen, insbesondere ist es vorteilhaft, wenn als physiologischen Sensor ein Hirndrucksensor verwendet wird, der dem Innendruck des Gehirns entsprechende Meßdaten liefert.

Der Erfindung liegt weiterhin die Aufgabe zugrunde, eine gattungsgemäße Vorrichtung so auszubilden, daß die Speicherung der von unterschiedlichen physiologischen Sensoren gelieferten Meßdaten durch die Auswerteeinheit vereinfacht wird.

Diese Aufgabe wird bei einer Vorrichtung der eingangs beschriebenen Art erfindungsgemäß durch die kennzeichnenden Merkmale von Anspruch 11 gelöst.

Die Auswerteeinheit ist insbesondere so ausgebildet, daß sie zur Überprüfung der Zuordnung eines Speichers zu einem physiologischen Sensor Codierungsdaten, die vom physiologischen Sensor der Auswerteeinheit zugeführt werden, und Codierungsdaten, die jeweils in jedem Speicher gespeichert sind, miteinander vergleicht und die vom physiologischen Sensor gelieferten Meßdaten nur dann in einem Speicher speichert, wenn die Codierungsdaten des physiologischen Sensors und des Speichers zueinander passen.

Bei einer bevorzugten Ausführungsform der Erfindung ist vorgesehen, daß jeder Speicher einen unveränderbaren Speicherbereich und einen veränderbaren Speicherbereich aufweist und daß die Codierungsdaten des Speichers in dem unveränderbaren Speicherbereich gespeichert sind.

Bei einer bevorzugten Weiterbildung der Erfindung kann vorgesehen sein, daß in den Speichern Kalibrationsdaten des zugeordneten physiologischen Sensors gespeichert sind und daß die Auswerteeinheit so ausgebildet ist, daß sie diese Kalibrationsdaten bei der Auswertung der Meßdaten berücksichtigt.

Besonders vorteilhaft ist es, wenn die verschiedenen Speicher als mobile, mit der Auswerteeinheit wahlweise verbindbare und wieder von ihr abtrennbare Baueinheiten ausgebildet sind, insbesondere können die Speicher als in die Auswerteeinheit einschiebbare Flash-Karten ausgebildet sein.

Bei einer weiteren Ausgestaltung der Erfindung ist vorgesehen, daß der Auswerteeinheit mindestens ein externer Sensor zugeordnet ist, der Umgebungsparametern entsprechende Meßdaten erzeugt, und daß die Auswerteeinheit so ausgebildet ist, daß sie diese Meßdaten zusammen mit den Meßdaten des physiologischen Sensors in dem dem physiologischen Sensor zugeordneten Speicher speichert.

Weiterhin ist es günstig, wenn die Auswerteeinheit so ausgebildet ist, daß sie die von einem physiologischen Sensor der Auswerteeinheit zugeführten Daten nur dann in dem zugeordneten Speicher speichert, wenn diese Meßdaten bestimmte Plausibilitätskriterien erfüllen.

Insbesondere kann dabei vorgesehen sein, daß die Auswerteeinheit so ausgebildet ist, daß sie Vergleichsdaten für die Überprüfung der Plausibilitätskriterien in dem den physiologischen Sensoren zugeordneten Speicher speichert.

Der physiologische Sensor kann beispielsweise ein Hirndrucksensor sein, der dem Innendruck des Gehirns entsprechende Meßdaten liefert.

Die nachfolgende Beschreibung bevorzugter Ausführungsformen der.Erfindung dient im Zusammenhang mit der Zeichnung der näheren Erläuterung. Es zeigen:

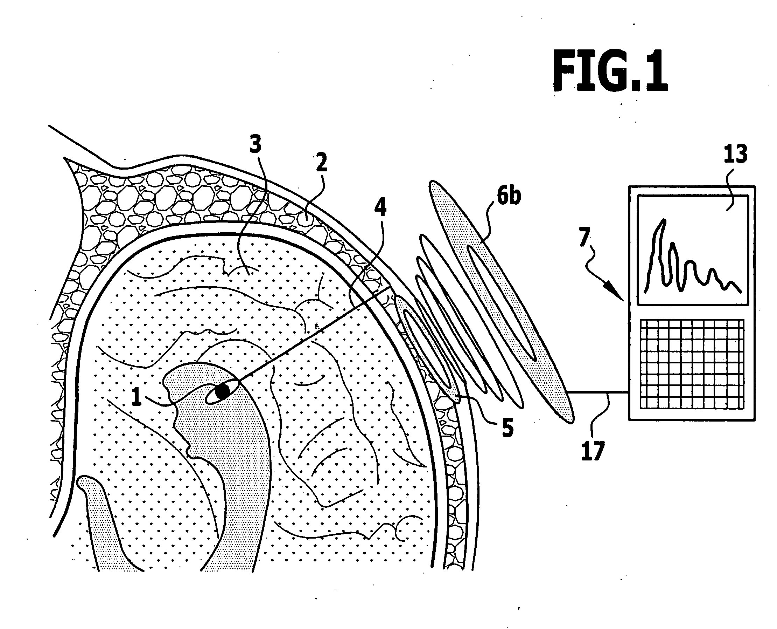

- Figur 1:: eine schematische Darstellung eines Hirndrucksensors und einer Meßdaten des Hirndrucksensors empfangenden Auswerteeinheit;

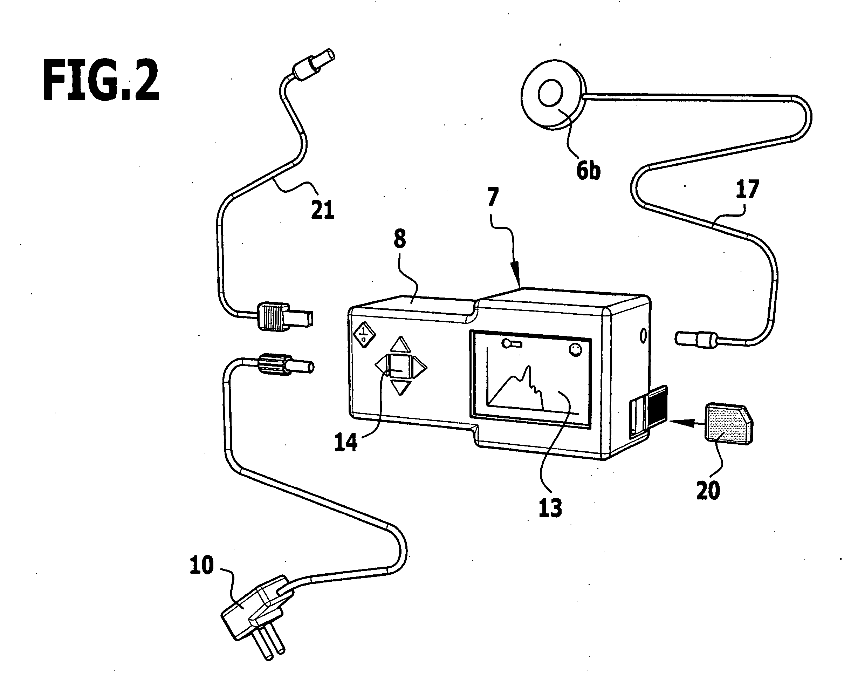

- Figur 2:: eine vereinfachte Darstellung einer Auswerteeinheit;

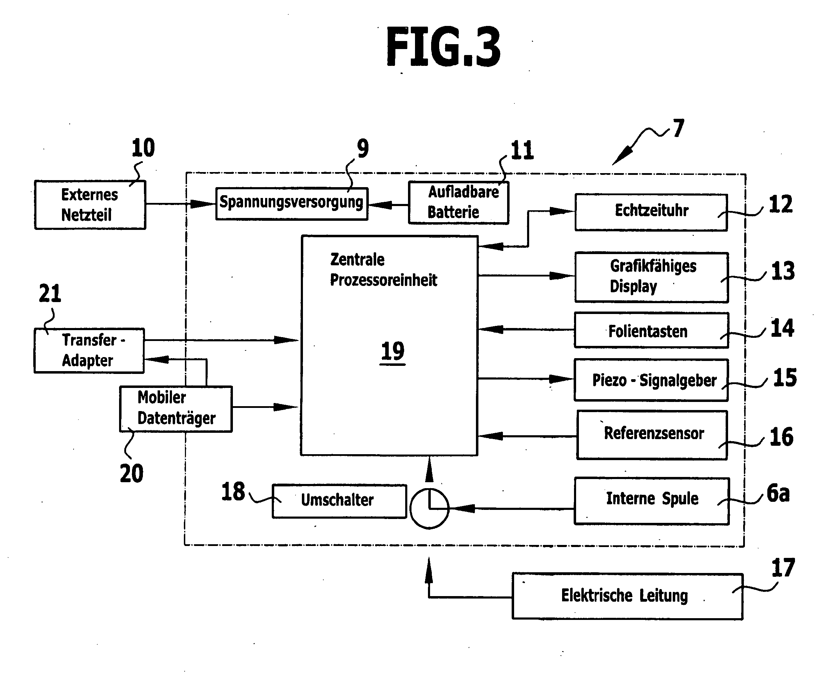

- Figur 3:: ein Blockschaltbild einer Auswerteeinheit;
- Figur 4:: ein Diagramm zur Darstellung der Druckamplitude des Hirninnen- drucks in Abhängigkeit vom durchschnittlichen Innendruck des Gehirns und
- Figur 5:: eine Darstellung von drei unterschiedlichen zeitlichen Verläufen der Hirndruckamplitude bei unterschiedlichen Hirnzuständen.

Die Erfindung wird am Beispiel eines Hirndrucksensors 1 erörtert, der durch die Schädeldecke 2 in das Hirn 3 eingeführt ist und vom Innendruck des Hirnes 3 abhängige elektrische Signale erzeugt, die über eine Leitung 4 zu einer auf die Außenseite der Schädeldecke 2 aufgelegten Übertragungsspule 5 geführt werden. Diese Übertragungsspule 5 überträgt diese Meßdaten drahtlos auf eine Empfängerspule 6a, die in einer Auswerteeinheit 7 angeordnet ist oder auf eine Empfängerspule 6b, die der Auswerteeinheit 7 über eine Leitung 17 in Verbindung steht. Die Auswerteeinheit 7 dient dazu, die empfangenen Meßdaten zu speichern und sie dem behandelnden Arzt unmittelbar oder nach Auswertung so anzuzeigen, daß die vom Hirndrucksensor 1 gelieferten Meßdaten Auskunft über die Meßgrößen geben, also im vorliegenden Beispiel über den Innendruck des Gehirns.

Die Auswerteeinheit ist vorzugsweise als selbständige Baueinheit in einem eigenen Gehäuse 8 untergebracht und verfügt über eine elektrische Spannungsversorgung 9, die entweder über ein externes Netzteil 10 oder über eine aufladbare Batterie 11 gespeist wird. Das Netzteil kann auch eine Ladestation sein.

An der Auswerteeinheit sind verschiedene Aggregate angeordnet, beispielsweise eine Echtzeituhr 12, ein graphikfähiges Display, beispielsweise in Form eines Flüssigkristall-Bildschirmes, Folientasten 14 zur Eingabe von Daten und Steuerbefehlen, ein Piezosignalgeber 15 zur Erzeugung der notwendigen Trägerfrequenz für die drahtlose Funkübertragung der Meßdaten von der Übertragungsspule 5 zur Empfängerspule 6, und ein Referenzsensor 16, mit dem externe Umgebungsparameter bestimmt werden können, beispielsweise der Umgebungsdruck.

Im dargestellten Ausführungsbeispiel werden die Meßdaten vom Hirndrucksensor 1 über die Leitung 4 drahtlos von der Übertragungsspule 5 auf die Empfängerspule 6a oder die Empfängerspule 6b übertragen. Bei einer anderen Ausgestaltung wäre es möglich, diese Datenübertragung leitungsgebunden über eine Datenleitung vorzunehmen, die dann eine unmittelbare Verbindung zwischen der Leitung 4 und der Auswerteeinheit 7 herstellen würde. Über einen in der Auswerteeinheit 7 angeordneten Umschalter 18 kann die Art der Meßdatenübermittlung ausgewählt werden, es ist also alternativ eine Umschaltung von drahtloser Übertragung über die Empfängerspule 6a auf galvanische Übertragung über die Datenleitung 17 möglich.

Die Datenleitung 17 kann aber auch mit einer von der Auswerteeinheit 7 entfernten Empfängerspule 6b verbunden sein, so daß dann der Umschalter 18 zwischen der Empfängerspule 6a in der Auswerteeinheit 7 und der über die Datenleitung 17 mit der Auswerteeinheit 7 in Verbindung stehenden Empfängerspule 6b umschaltet.

Alle genannten Aggregate stehen mit einer zentralen Prozessoreinheit 19 der Auswerteeinheit 7 in Verbindung, in welcher die von den Aggregaten erzeugten und die über die Empfängerspule 6 oder die Datenleitung 17 zugeführten Daten verarbeitet werden.

In die Auswerteeinheit 7 ist über einen seitlichen Steckplatz eine mobile Speicherkarte 20 einschiebbar, die beispielsweise als an sich bekannte Flash-Karte ausgebildet sein kann. Im eingeschobenen Zustand steht diese mobile Speicherkarte 20 mit der zentralen Prozessoreinheit 19 in Verbindung, so daß die zentrale Prozessoreinheit Daten auf der mobilen Speicherkarte abspeichern und bei Bedarf auch auslesen kann.

Die zentrale Prozessoreinheit und gegebenenfalls auch die mobile Speicherkarte 20 können zusätzlich über eine Datenleitung 21 mit einem externen Datenverarbeitungsgerät verbunden werden, beispielsweise einem Computer, in dem die in der Auswerteeinheit empfangenen und in der mobilen Speicherkarte 20 gespeicherten Daten weiterverarbeitet werden können.

Die mobile Speicherkarte 20 ist in zwei Speicherbereiche unterteilt, nämlich einen unveränderbaren Speicherbereich, in dem Daten dauerhaft gespeichert sind, und in einen beschreibbaren Speicherbereich, in dem die Auswerteeinheit Daten speichern kann. Die Daten beider Bereiche können von der zentralen Prozessoreinheit ausgelesen werden.

In dem unveränderbaren Speicherbereich einer mobilen Speicherkarte 20 ist eine Codierung dauerhaft gespeichert, durch die die spezielle mobile Speicherkarte 20 identifizierbar ist.

Eine ähnliche Identifikations-Codierung ist auch im Hirndrucksensor 1 gespeichert, und der Hirndrucksensor 1 sendet zusammen mit den den physiologischen Parametern entsprechenden Meßdaten auch Daten an die Auswerteeinheit 7, die dieser Identifikations-Codierung entsprechen.

Die zentrale Prozessoreinheit vergleicht die von dem Hirndrucksensor 1 zugeführten Codierungen mit den Codierungen der jeweils eingesetzten mobilen Speicherkarte 20 und gibt diese mobile Speicherkarte 20 zur Speicherung von Meßdaten nur dann frei, wenn die Codierungen des Hirndrucksensors 1 und der mobilen Speicherkarte 20 zueinander passen. Diese Passung kann eine Identität sein, es ist aber auch möglich, daß die Übereinstimmung nach verschiedenen Kriterien bestimmt wird. Wesentlich ist lediglich, daß zu einem Hirndrucksensor 1 nur eine einzige mobile Speicherkarte 20 verwendet wird, die eine passende Codierung aufweist. Umgekehrt gibt es auch für jede mobile Speicherkarte 20 nur einen einzigen Hirndrucksensor 1 mit passender Codierung, es besteht also eine eindeutige Zuordnung zwischen Hirndrucksensor 1 und mobiler Speicherkarte 20.

Natürlich ist es möglich, daß der Hersteller als Ersatz eine weitere mobile Speicherkarte 20 mit identischer Codierung im Vorrat hat, diese darf aber nur bei Verlust oder dauerhafter Beschädigung der aktuellen Speicherkarte 20 eingesetzt werden, so daß sichergestellt ist, daß die Speicherung der von einem physiologischen Sensor stammenden Daten immer nur auf einer einzigen Speicherkarte 20 erfolgt.

Die Auswerteeinheit ist damit nur dann in der Lage, die empfangenen Meßdaten eines Hirndrucksensors 1 zu speichern, wenn die zugeordnete mobile Speicherkarte 20 in die Auswerteeinheit 7 eingeschoben ist, beim Fehlen einer solchen Speicherkarte oder beim Einsetzen einer anderen Speicherkarte erfolgt keine Speicherung der Meßdaten, dagegen kann dann ein Warnsignal erzeugt werden, daß den Benutzer darauf aufmerksam macht, daß die zu dem Hirndrucksensor 1 passende Speicherkarte nicht eingeschoben ist.

Damit ist sichergestellt, daß die von einem Hirndrucksensor stammenden Meßdaten immer nur auf derselben, zugeordneten mobilen Speicherkarte 20 gespeichert werden und daß keine Verwechslungen auftreten können. Zusätzliche Eingaben mit Patientendaten etc. sind daher nicht notwendig.

Auf der mobilen Speicherkarte 20 können weitere Daten gespeichert werden, beispielsweise die von der Echtzeituhr 12 gelieferten Zeitdaten oder Meßdaten des Referenzsensors 16. Auch in diesem Falle werden die Daten auf die mobile Speicherkarte 20 gespeichert, die dem gerade angeschlossenen Hirndrucksensor 1 entspricht. Damit werden auf dieser Speicherkarte beispielsweise die momentanen Hirndruckwerte und gleichzeitig die externen Umgebungsdruckwerte gespeichert, so daß die Auswerteeinheit oder eine angeschlossene Recheneinheit den Differenzdruck jederzeit bestimmen können.

Weiterhin können - vorzugsweise im unveränderbaren Speicherbereich - Kalibrationsdaten des Hirndrucksensors 1 abgespeichert sein, durch die typische Kenngrößen des Hirndrucksensors 1 definiert werden, beispielsweise die Empfindlichkeit, eine Temperaturabhängigkeit oder andere spezifische Größen, die bei der Auswertung der Meßdaten des Hirndrucksensors 1 von Bedeutung sein können. Diese für den angeschlossenen Hirndrucksensor 1 spezifischen Kenngrößen können auf diese Weise berücksichtigt werden, da sie auf der zugeordneten mobilen Speicherkarte 20 gespeichert sind. Verwechslungen sind auch in diesem Falle nicht möglich, außerdem ist es für den Benutzer nicht notwendig, spezifische Kenngrößen des angeschlossenen Hirndrucksensors 1 von Hand in die Auswerteeinheit 7 einzugeben. Dadurch wird die Sicherheit des Betriebes erheblich erhöht.

Bei einer bevorzugten Weiterbildung sind auf der jeweiligen mobilen Speicherkarte 20 oder aber in der zentralen Prozessoreinheit zusätzlich Plausibilitätskriterien gespeichert, die von der zentralen Prozessoreinheit mit den empfangenen Meßdaten verglichen werden. Wenn die empfangenen Meßdaten von diesen Plausibilitätskriterien in einem vorgegebenen Toleranzbereich abweichen, werden die Meßdaten als echte Meßdaten gewertet und in der beschriebenen Weise auf der mobilen Speicherkarte 20 abgelegt, wenn dagegen die Abweichungen größer sind, als es durch den vorgegebenen Toleranzbereich erwartet wird, wird die Speicherung dieser Meßdaten verhindert, und es wird ein Warnsignal ausgegeben. Mit anderen Worten, in einem solchen Fall werden die vom Hirndrucksensor 1 gelieferten Meßdaten nicht als Meßwerte übernommen, sondern dienen lediglich der Anzeige, daß Meßwerte vom Hirndrucksensor 1 geliefert werden, die nicht den Plausibilitätskriterien entsprechen. Dies kann verursacht werden durch eine Fehlfunktion des Hirndrucksensors 1 oder aber auch durch einen anomalen Zustand des Patienten. In beiden Fällen sind zusätzliche Kontrollmaßnahmen notwendig, die vom Arzt aufgrund des Warnsignales durchgeführt werden können.

Auch beim Abweichen von den Plausibilitätskriterien kann allerdings vorgesehen sein, daß die vom physiologischen Sensor gelieferten Daten gespeichert werden, damit ein Arzt auch in diesem Falle später kontrollieren kann, ob die Abweichung von den Plausibilitätskriterien apparativ bedingt ist oder ob tatsächlich physiologische Effekte dafür verantwortlich sind, denen der Arzt dann in jedem Fall nachgehen muß. Es ist aber dann vorzugsweise vorgesehen, daß die entsprechenden Daten auf der Speicherkarte gekennzeichnet werden, so daß der Arzt beim Betrachten der Daten sofort erkennen kann, daß bei diesen Daten eine Abweichung von den Plausibilitätskriterien vorgelegen hat.

Die Plausibilitätskriterien können beispielsweise bestimmte Wertebereiche sein, innerhalb welcher sich die Meßwerte befinden müssen, es können auch kompliziertere Zusammenhänge untersucht werden, um geeignete Plausibilitätskriterien zu finden.

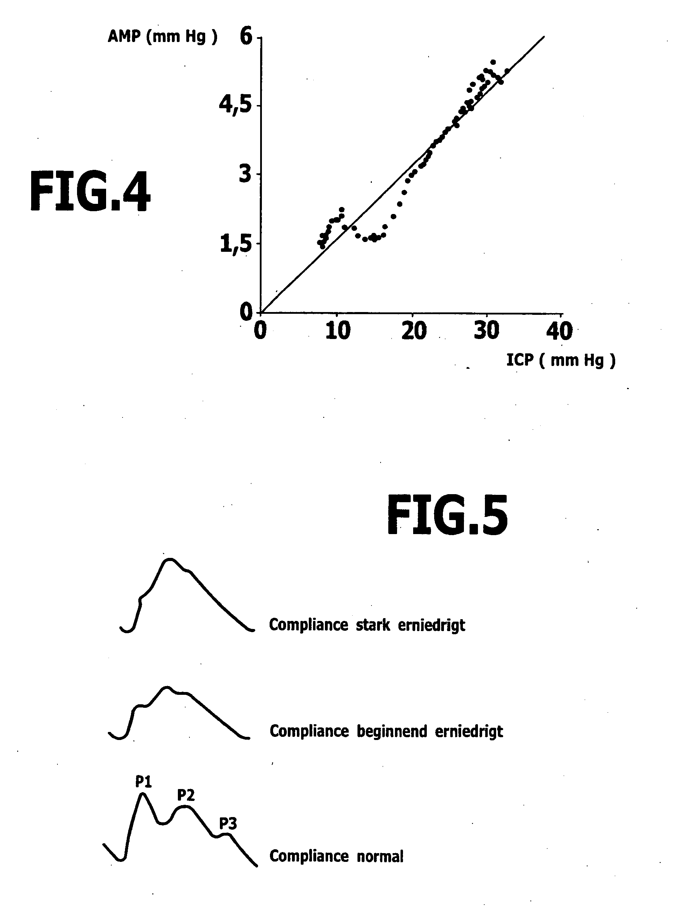

Bei der Hirndruckmessung ist es beispielsweise möglich, ein solches Plausibilitätskriterium aus einem Quotienten zu bestimmen, der die Amplitude eines Druckimpulses im Hirn vergleicht mit dem Mittelwert des Hirndruckes. Dieser Quotient liegt normalerweise bei etwa 0,2, und Abweichungen, die über eine vorbestimmte Toleranz hinausgehen, sind ein Zeichen dafür, daß der Hirndrucksensor 1 nicht zuverlässig arbeitet oder das Gehirn in einem anormalen Zustand ist. In Figur 4 ist ein Diagramm dargestellt, bei dem mehrere Messungen der Druckamplitude und des durchschnittlichen Druckes in einem Diagramm eingetragen sind, diese Meßpunkte können durch eine Gerade angenähert werden, die etwa die gewünschte Steigung von 0,2 aufweist, dies entspricht dem Normalzustand bei funktionsfähigem Hirndrucksensor und normal eingestelltem Patienten. Größere Abweichungen würden feststellbar sein und zu den genannten Abhilfereaktionen führen.

Ein weiteres Kriterium ist dabei der Durchschnittswert des Hirninnendrucks selbst, dieser sollte zwischen 20 und 60 mm Hg liegen. Auch dies kann als Plausibilitätskriterium überprüft werden. Ein weiteres Plausibilitätskriterium läßt sich aus dem zeitlichen Verlauf des Druckimpulses im Gehirn bestimmen. Der Hirninnendruck wird bestimmt durch arterielle Zuströme und venöse Abströme, und dabei erhält man pulssynchrone Schwankungen des Hirninnendrucks. Normalerweise wird die Hirndruckkurve, also der zeitliche Druckverlauf, durch mehrere Einzelspitzen gekennzeichnet, in Figur 5 ist in der unteren Darstellung ein solcher Verlauf mit drei Einzelspitzen P1, P2 und P3 dargestellt.

Wenn der Zustand des Gehirns abweicht von dem Normalzustand, werden die Einzelspitzen zunehmend undeutlicher und verschwinden schließlich ganz, wie dies bei der mittleren, bzw. der oberen Kurve in Figur 5 dargestellt ist. Ein Vergleich der gemessenen Kurvenform mit Kurvenformen, wie sie in Figur 5 dargestellt sind, kann daher als Plausibilitätskriterium dafür dienen, ob der Zustand des Gehirns normal ist oder ob Abweichungen auftreten, die dazu führen, daß gemessene Meßdaten nicht auf der mobilen Speicherkarte gespeichert werden, sondern daß ein Warnsignal für den Arzt ausgegeben wird.

Die Verwendung einer mobilen Speicherkarte, die einem bestimmten Hirndrucksensor 1 zugeordnet ist, macht es einerseits möglich, mit einer Auswerteeinheit die Meßdaten von verschiedenen Hirndrucksensoren 1 zu erfassen und ausschließlich auf der zugeordneten Speicherkarte abzulegen, andererseits ist es auch möglich, daß ein Patient mit einem Hirndrucksensor 1 die entsprechenden Meßdaten bei verschiedenen Auswerteeinheiten abfragen läßt, die dann auf die mitgeführte mobile Speicherkarte 20 die entsprechenden Meßdaten ablegen können. In jedem Fall ist eine sichere Zuordnung gewährleistet, und der Bedienungskomfort ist gegenüber herkömmlichen Systemen, bei denen Patientendaten von Hand eingegeben werden müssen, verbessert.

## Patentansprüche

1. Verfahren zur Datenerfassung von Meßdaten, die von einem physiologischen Sensor in Abhängigkeit von physiologischen Parametern eines Lebewesens erzeugt werden, bei dem die von dem physiologischen Sensor erzeugten Daten einer externen Auswerteeinheit zugeführt werden, die diese Daten speichert, **dadurch gekennzeichnet, daß** die Auswerteeinheit dazu ausgebildet ist, mit verschiedenen physiologischen Sensoren zusammenzuarbeiten, jedem physiologischen Sensor ein eigener Speicher zugeordnet ist und die Auswerteeinheit dazu ausgebildet ist, von einem physiologischen Sensor stammende Daten ausschließlich in dem diesem physiologischen Sensor zugeordneten Speicher zu speichern.

2. Verfahren nach Anspruch 1, **dadurch gekennzeichnet, daß** die Auswerteeinheit zur Überprüfung der Zuordnung eines Speichers zu einem physiologischen Sensor Codierungsdaten, die vom physiologischen Sensor der Auswerteeinheit zugeführt werden, und Codierungsdaten, die jeweils in jedem Speicher gespeichert sind, miteinander vergleicht und die vom physiologischen Sensor gelieferten Meßdaten nur dann in einem Speicher speichert, wenn die Codierungsdaten des physiologischen Sensors und des Speichers zueinander passen.

3. Verfahren nach Anspruch 2, **dadurch gekennzeichnet, daß** die Codierungsdaten in dem Speicher unveränderbar gespeichert werden.

4. Verfahren nach Anspruch 2 oder 3, **dadurch gekennzeichnet, daß** in den Speichern Kalibrationsdaten des zugeordneten physiologischen Sensors gespeichert werden und daß die Auswerteeinheit diese bei der Auswertung der Meßdaten berücksichtigt.

5. Verfahren nach einem der voranstehenden Ansprüche, **dadurch gekennzeichnet, daß** die verschiedenen jeweils einem physiologischen Sensor zugeordneten Speicher als mobile Baueinheiten ausgebildet und diese zur Datenerfassung der Meßdaten eines physiologischen Sensors jeweils mit der Auswerteeinheit verbunden werden.

6. Verfahren nach Anspruch 5, **dadurch gekennzeichnet, daß** als Speicher in die Auswerteeinheit einschiebbare Flash-Karten verwendet werden.

7. Verfahren nach einem der voranstehenden Ansprüche, **dadurch gekennzeichnet, daß** bei der Datenerfassung von Meßdaten des physiologischen Sensors zusätzlich mittels eines externen Sensors Meßdaten erzeugt werden, die Umgebungsparametern entsprechen, und daß diese Meßdaten zusammen mit den Meßdaten des physiologischen Sensors in dem diesem physiologischen Sensor zugeordneten Speicher gespeichert werden.

8. Verfahren nach einem der voranstehenden Ansprüche, **dadurch gekennzeichnet, daß** die Auswerteeinheit die von einem physiologischen Sensor der Auswerteeinheit zugeführten Meßdaten nur dann in dem zugeordneten Speicher speichert, wenn diese Meßdaten bestimmte Plausibilitätskriterien erfüllen.

9. Verfahren nach Anspruch 8, **dadurch gekennzeichnet, daß** Vergleichsdaten für die Überprüfung der Plausibilitätskriterien in den den physiologischen Sensoren zugeordneten Speichern gespeichert werden.

10. Verfahren nach einem der voranstehenden Ansprüche, **dadurch gekennzeichnet, daß** als physiologischer Sensor ein Hirndrucksensor verwendet wird, der dem Innendruck des Gehirns entsprechende Meßdaten liefert.

11. Vorrichtung zur Datenerfassung von Meßdaten, die von einem physiologischen Sensor (1) in Abhängigkeit von physiologischen Parametern eines Lebewesens erzeugt werden, mit einer Auswerteeinheit (7), die über eine Datenübertragungsstrecke mit dem physiologischen Sensor (1) verbindbar ist, und mit einer Speichereinrichtung, in der die Auswerteeinheit (7) die von dem physiologischen Sensor (1) gelieferten Meßdaten speichert, **dadurch gekennzeichnet, daß** die Answerteeinheit dazu ausgebildet ist, mit verschiedenen physiologischen Sensoren zusammenzuarbeiten, und die Speichereinrichtung für jeden physiologischen Sensor (1) einen eigenen Speicher (20) aufweist, in dem die Auswerteeinheit (7) von dem physiologischen Sensor (1) stammende Meßdaten ausschließlich speichert.

12. Vorrichtung nach Anspruch 11, **dadurch gekennzeichnet, daß** die Auswerteeinheit (7) so ausgebildet ist, daß sie zur Überprüfung der Zuordnung eines Speichers (20) zu einem physiologischen Sensor (1) Codierungsdaten, die vom physiologischen Sensor (1) der Auswerteeinheit (7) zugeführt werden, und Codierungsdaten, die jeweils in jedem Speicher (20) gespeichert sind, miteinander vergleicht und die vom physiologischen Sensor (1) gelieferten Meßdaten nur dann in einem Speicher (20) speichert, wenn die Codierungsdaten des physiologischen Sensors (1) und des Speichers (20) zueinander passen.

13. Vorrichtung nach Anspruch 12, **dadurch gekennzeichnet, daß** jeder Speicher (20) einen unveränderbaren Speicherbereich und einen veränderbaren Speicherbereich aufweist und daß die Codierungsdaten des Speichers (20) in dem unveränderbaren Speicherbereich gespeichert sind.

14. Vorrichtung nach einem der Ansprüche 12 oder 13, **dadurch gekennzeichnet, daß** in den Speichern (20) Kalibrationsdaten des zugeordneten physiologischen Sensors (1) gespeichert sind und daß die Auswerteeinheit (7) so ausgebildet ist, daß sie diese Kalibrationsdaten bei der Auswertung der Meßdaten berücksichtigt.

15. Vorrichtung nach einem der Ansprüche 11 bis 14, **dadurch gekennzeichnet, daß** die verschiedenen Speicher als mobile, mit der Auswerteeinheit (7) wahlweise verbindbare und wieder von ihr abtrennbare Baueinheiten ausgebildet sind.

16. Vorrichtung nach Anspruch 15, **dadurch gekennzeichnet, daß** die Speicher (20) als in die Auswerteeinheit (7) einschiebbare Flash-Karten ausgebildet sind.

17. Vorrichtung nach einem der Ansprüche 11 bis 16, **dadurch gekennzeichnet, daß** der Auswerteeinheit (7) mindestens ein externer Sensor (16) zugeordnet ist, der Umgebungsparametern entsprechende Meßdaten erzeugt, und daß die Auswerteeinheit (7) so ausgebildet ist, daß sie diese Meßdaten zusammen mit den Meßdaten eines physiologischen Sensors (1) in dem dem physiologischen Sensor (1) zugeordneten Speicher (20) speichert.

18. Vorrichtung nach einem der Ansprüche 11 bis 17, **dadurch gekennzeichnet, daß** die Auswerteeinheit (7) so ausgebildet ist, daß sie die von einem physiologischen Sensor (1) der Auswerteeinheit (7) zugeführten Meßdaten nur dann in dem zugeordneten Speicher (20) speichert, wenn diese Meßdaten bestimmte Plausibilitätskriterien erfüllen.

19. Vorrichtung nach Anspruch 18, **dadurch gekennzeichnet, daß** die Vergleichsdaten für die Überprüfung der Plausibilitätskriterien in dem den physiologischen Sensoren (1) zugeordneten Speicher (20) gespeichert sind.

20. Vorrichtung nach einem der Ansprüche 11 bis 19, **dadurch gekennzeichnet, daß** die physiologischen Sensoren (1) ein Hirndrucksensoren sind, die dem Innendruck des Gehirns entsprechende Meßdaten liefern.

## Claims

1. Method for acquiring measurement data which are generated by a physiological sensor as a function of physiological parameters of a living being, in which the data generated by the physiological sensor are supplied to an external evaluation unit, which stores these data, **characterised in that** the evaluation unit is configured to cooperate with various physiological sensors, each physiological sensor has its own memory associated with it, and the evaluation unit is configured to store data originating from a physiological sensor exclusively in the memory associated with this physiological sensor.

2. Method according to claim 1, **characterised in that**, to check the association of a memory with a physiological sensor, the evaluation unit compares coding data, which are supplied to the evaluation unit by the physiological sensor, and coding data, which are in each case stored in each memory, with one another and only stores the measurement data supplied by the physiological sensor in a memory if the coding data of the physiological sensor and the memory match one another.

3. Method according to claim 2, **characterised in that** the coding data are stored in the memory in a non-variable manner.

4. Method according to claim 2 or 3, **characterised in that** calibration data of the associated physiological sensor are stored in the memories, and **in that** the evaluation unit takes these into account when evaluating the measurement data.

5. Method according to any one of the preceding claims, **characterised in that** the various memories, each associated with a physiological sensor, are configured as mobile assemblies, and these are connected in each case to the evaluation unit to acquire the measurement data of a physiological sensor.

6. Method according to claim 5, **characterised in that** flash cards which can be inserted in the evaluation unit are used as memories.

7. Method according to any one of the preceding claims, **characterised in that** when acquiring measurement data of the physiological sensor, measurement data corresponding to environmental parameters are additionally generated by means of an external sensor, and **in that** these measurement data are stored together with the measurement data of the physiological sensor in the memory associated with this physiological sensor.

8. Method according to any one of the preceding claims, **characterised in that** the evaluation unit only stores the measurement data supplied by a physiological sensor to the evaluation unit in the associated memory if these measurement data satisfy certain plausibility criteria.

9. Method according to claim 8, **characterised in that** comparison data for checking the plausibility criteria are stored in the memories associated with the physiological sensors.

10. Method according to any one of the preceding claims, **characterised in that** an intracranial pressure sensor, which supplies measurement data corresponding to the internal pressure of the brain, is used as physiological sensor.

11. Device for acquiring measurement data, which are generated by a physiological sensor (1) as a function of physiological parameters of a living being, comprising an evaluation unit (7), which is connectable to the physiological sensor (1) by means of a data transmission path, and comprising a memory device, in which the evaluation unit (7) stores the measurement data supplied by the physiological sensor (1), **characterised in that** the evaluation unit is configured to cooperate with various physiological sensors, and the memory device comprises for each physiological sensor (1) its own memory (20), in which the evaluation unit (7) exclusively stores measurement data originating from the physiological sensor (1).

12. Device according to claim 11, **characterised in that** the evaluation unit (7) is configured in such a way that, to check the association of a memory (20) with a physiological sensor (1), it compares coding data, which are supplied by the physiological sensor (1) to the evaluation unit (7), and coding data, which are stored in each case in each memory (20), with one another, and only stores the measurement data supplied by the physiological sensor (1) in a memory (20) if the coding data of the physiological sensor (1) and the memory (20) match one another.

13. Device according to claim 12, **characterised in that** each memory (20) has a non-variable memory region and a variable memory region, and **in that** the coding data of the memory (20) are stored in the non-variable memory region.

14. Device according to either of claims 12 or 13, **characterised in that** calibration data of the associated physiological sensor (1) are stored in the memories (20), and **in that** the evaluation unit (7) is configured in such a way that it takes into account these calibration data when evaluating the measurement data.

15. Device according to any one of claims 11 to 14, **characterised in that** the various memories are configured as mobile assemblies which can selectively be connected to the evaluation unit (7) and separated again therefrom.

16. Device according to claim 15, **characterised in that** the memories (20) are configured as flash cards which can be inserted in the evaluation unit (7).

17. Device according to any one of claims 11 to 16, **characterised in that** at least one external sensor (16) is associated with the evaluation unit (7) and generates measurement data corresponding to environmental parameters, and **in that** the evaluation unit (7) is configured in such a way that it stores these measurement data together with the measurement data of a physiological sensor (1) in the memory (20) associated with the physiological sensor (1).

18. Device according to any one of claims 11 to 17, **characterised in that** the evaluation unit (7) is configured in such a way that it only stores the measurement data supplied by a physiological sensor (1) to the evaluation unit (7) in the associated memory (20) if these measurement data satisfy certain plausibility criteria.

19. Device according to claim 18, **characterised in that** the comparison data for checking the plausibility criteria are stored in the memory (20) associated with the physiological sensors (1).

20. Device according to any one of claims 11 to 19, **characterised in that** the physiological sensors (1) are intracranial pressure sensors, which supply measurement data corresponding to the internal pressure of the brain.

## Revendications

1. Procédé de relevé de données de données de mesure, qui sont produites par un capteur physiologique en fonction de paramètres physiologiques d'un être vivant, selon lequel on transmet les données produites par le capteur physiologique à une unité de traitement de données externe, qui mémorise ces données,
**caractérisé en ce que** l'unité de traitement de données est conçue pour coopérer avec des capteurs physiologiques différents, **en ce qu'**à chaque capteur physiologique est affectée sa propre une mémoire, et **en ce que** l'unité de traitement de données est conçue pour mémoriser les données, qui proviennent d'un capteur physiologique, exclusivement dans la mémoire affectée à ce capteur physiologique.

2. Procédé selon la revendication 1, **caractérisé en ce que** l'unité de traitement de données, pour vérifier l'affectation d'une mémoire à un capteur physiologique, compare réciproquement des données de codage, qui sont transmises par le capteur physiologique à l'unité de traitement de données, et des données de codage, qui sont mémorisées respectivement dans chaque mémoire, et mémorise les données de mesure fournies par le capteur physiologique, dans une mémoire, uniquement si les données de codage du capteur physiologique et de la mémoire sont mutuellement adaptées.

3. Procédé selon la revendication 2, **caractérisé en ce que** l'on mémorise les données de codage de manière non modifiable dans la mémoire.

4. Procédé selon la revendication 2 ou la revendication 3, **caractérisé en ce que** l'on mémorise dans les mémoires, des données de calibrage du capteur physiologique qui leur est affecté, et **en ce que** l'unité de traitement de données prend celles-ci en considération lors du traitement des données de mesure.

5. Procédé selon l'une des revendications précédentes, **caractérisé en ce que** les différentes mémoires respectivement affectées à un capteur physiologique, sont réalisées en tant que modules mobiles, et l'on relie celles-ci respectivement à l'unité de traitement de données pour le relevé de données des données de mesure d'un capteur physiologique.

6. Procédé selon la revendication 5, **caractérisé en ce que** l'on utilise en guise de mémoires, des cartes mémoire-flash pouvant être insérées dans l'unité de traitement de données.

7. Procédé selon l'une des revendications précédentes, **caractérisé en ce que** lors du relevé de données de données de mesure du capteur physiologique, on produit en supplément, à l'aide d'un capteur externe, des données de mesure qui correspondent à des paramètres d'environnement, et **en ce que** l'on mémorise ces données de mesure en commun avec les données de mesure du capteur physiologique, dans la mémoire affectée à ce capteur physiologique.

8. Procédé selon l'une des revendications précédentes, **caractérisé en ce que** l'unité de traitement de données mémorise les données de mesure transmises par un capteur physiologique à l'unité de traitement de données, dans la mémoire qui lui est affectée, uniquement si ces données de mesure remplissent des critères de plausibilité déterminés.

9. Procédé selon la revendication 8, **caractérisé en ce que** l'on mémorise dans les mémoires affectées aux capteurs physiologiques, des données de comparaison pour la vérification des critères de plausibilité.

10. Procédé selon l'une des revendications précédentes, **caractérisé en ce que** l'on met en oeuvre en tant que capteur physiologique, un capteur de pression intracrânienne, qui fournit des données de mesure correspondant à la pression intérieure de la cavité encéphalique.

11. Dispositif pour le relevé de données de données de mesure, qui sont produites par un capteur physiologique (1) en fonction de paramètres physiologiques d'un être vivant, comprenant une unité de traitement de données (7) qui peut être reliée au capteur physiologique (1) par l'intermédiaire d'un parcours de transmission de données, et comprenant également un système de mémoire dans lequel l'unité de traitement de données (7) mémorise les données de mesure fournies par le capteur physiologique (1),
**caractérisé en ce que** l'unité de traitement de données est conçue pour coopérer avec des capteurs physiologiques différents, et le système de mémoire présente, pour chaque capteur physiologique (1), une mémoire (20) qui lui est propre, dans laquelle l'unité de traitement de données (7) mémorise exclusivement des données de mesure provenant du capteur physiologique (1).

12. Dispositif selon la revendication 11, **caractérisé en ce que** l'unité de traitement de données (7) est conçue de façon telle que, pour vérifier l'affectation d'une mémoire (20) à un capteur physiologique (1), elle compare réciproquement des données de codage, qui sont transmises par le capteur physiologique (1) à l'unité de traitement de données (7), et des données de codage, qui sont mémorisées respectivement dans chaque mémoire (20), et mémorise les données de mesure fournies par le capteur physiologique (1), dans une mémoire (20), uniquement si les données de codage du capteur physiologique (1) et de la mémoire (20) sont mutuellement adaptées.

13. Dispositif selon la revendication 12, **caractérisé en ce que** chaque mémoire (20) présente une zone de mémoire non modifiable et une zone de mémoire modifiable, et **en ce que** les données de codage de la mémoire (20) sont mémorisées dans la zone de mémoire non modifiable.

14. Dispositif selon l'une des revendications 12 ou 13, **caractérisé en ce que** dans les mémoires (20) sont mémorisées des données de calibrage du capteur physiologique (1) qui leur est affecté, et **en ce que** l'unité de traitement de données (7) est conçue de manière à prendre en considération ces données de calibrage lors du traitement des données de mesure.

15. Dispositif selon l'une des revendications 11 à 14, **caractérisé en ce que** les différentes mémoires sont réalisées sous la forme de modules mobiles, qui peuvent être reliés sélectivement à l'unité de traitement de données (7) et à nouveau en être séparés.

16. Dispositif selon la revendication 15, **caractérisé en ce que** les mémoires (20) sont réalisées en tant que cartes mémoire-flash pouvant être insérées dans l'unité de traitement de données (7).

17. Dispositif selon l'une des revendications 11 à 16, **caractérisé en ce qu'**à l'unité de traitement de données (7) est associé au moins un capteur externe (16) produisant des données de mesure correspondant à des paramètres de l'environnement, et **en ce que** l'unité de traitement de données (7) est conçue pour mémoriser ces données de mesure en commun avec les données de mesure d'un capteur physiologique (1), dans la mémoire (20) affectée au capteur physiologique (1).

18. Dispositif selon l'une des revendications 11 à 17, **caractérisé en ce que** l'unité de traitement de données (7) est conçue pour mémoriser les données de mesure transmises par un capteur physiologique (1) à l'unité de traitement de données (7), dans la mémoire (20) qui lui est affectée, uniquement si ces données de mesure remplissent des critères de plausibilité déterminés.

19. Dispositif selon la revendication 18, **caractérisé en ce que** les données de comparaison pour la vérification des critères de plausibilité, sont mémorisées dans la mémoire (20) affectée aux capteurs physiologiques (1).

20. Dispositif selon l'une des revendications 11 à 19, **caractérisé en ce que** les capteurs physiologiques (1) sont des capteurs de pression intracrânienne, qui fournissent des données de mesure correspondant à la pression intérieure de la cavité encéphalique.
